# EUROPÄISCHE PATENTSCHRIFT

(11) **EP 2 670 706 B1**
(45) Veröffentlichungstag und Bekanntmachung des Hinweises auf die Patenterteilung: **07.08.2019**
(21) Anmeldenummer: 12703742.2
(22) Anmeldetag: 31.01.2012
(51) Int. Cl.: A61L 26/00, A61L 27/02, A61L 27/48, C01B 33/145, C01B 33/148

(54) **KIESELSÄUREKONDENSATE MIT GERINGER VERNETZUNG**
SILICIC ACID CONDENSATES HAVING A LOW CROSS-LINKAGE RATE
CONDENSATS D'ACIDE SILICIQUE À FAIBLE RÉTICULATION

(30) Priorität: 31.01.2011 DE 102011009839; 31.01.2011 DE 102011009838
(43) Veröffentlichungstag der Anmeldung: 11.12.2013
(73) Patentinhaber: Gerber, Thomas, 18059 Sildemow (DE)
(72) Erfinder: Gerber, Thomas, 18059 Sildemow (DE)
(74) Vertreter: Uexküll & Stolberg
(86) Internationale Anmeldenummer: PCT/EP2012/051603
(87) Internationale Veröffentlichungsnummer: WO 2012/104320

(56) Entgegenhaltungen:
- EP-A1- 0 881 192
- WO-A2-01/52618
- WO-A2-2005/087284
- WO-A2-2009/030919
- PUPUTTI J ET AL: "Stabilization of silica sols derived from an inexpensive, fully inorganic precursor and their use", JOURNAL OF NON-CRYSTALLINE SOLIDS, NORTH-HOLLAND PHYSICS PUBLISHING. AMSTERDAM, NL, Bd. 342, Nr. 1-3, 15. August 2004 (2004-08-15), Seiten 59-64, XP004560759, ISSN: 0022-3093, DOI: 10.1016/J.JNONCRYSOL.2004.06.016

## Beschreibung

Gegenstand der Erfindung ist ein Biomaterial, umfassend Kieselsäurekondensate mit geringer Vernetzung, und Verfahren zu dessen Herstellung. Es wird ein Verfahren zur Herstellung gering vernetzter Kieselsäurestrukturen offenbart, bei dem man ein Sol erzeugt, wobei man eine weitere Kondensation verhindert, wenn bestimmte Vernetzungen der Kieselsäure erreicht werden. Eine weitere Kondensation wird durch eine Veresterung von OH-Gruppen der Kieselsäurekondensate verhindert. In einer Ausführungsform umfasst das Material vorwiegend Siliziumdioxid (SiO₂), insbesondere solches, das wegen der Veresterung nanostrukturiert und gering vernetzt ist. Das Material kann in der Medizin für therapeutische Zwecke oder für kosmetische Zwecke eingesetzt werden und dabei einen unmittelbaren Kontakt mit biologischem Gewebe des Körpers eingehen. Dieses Material tritt dabei in chemische, physikalische und biologische Wechselwirkungen mit den entsprechenden biologischen Systemen. Es kann dabei abgebaut werden und als Lieferant für die im Stoffwechsel benötigte Kieselsäure wirken. Es kann darüber hinaus eine stützende oder abschirmende Wirkung haben. Es kann als Granulat, Mikropartikel, Faser und daraus hergestelltes Gewebe oder Vlies oder als Schicht auf Implantaten oder Wundverbänden vorliegen. Das Material kann als Medizinprodukt oder als Nahrungsergänzung verwendet werden.

Seit den 70iger Jahren des letzten Jahrhunderts ist bekannt, dass Silizium ein wichtiges Spurenelement für den Aufbau von Knochen und Kollagen ist. (siehe z.B. M. Carlisle; Silicon: An Essential Element for the Chick; Science 10 November 1972:Vol. 178. no. 4061, pp. 619 - 621). Die genauen biochemischen Prozesse sind nach wie vor nicht bekannt. Im Stoffwechsel kommt Silizium in erster Linie als Silizumdioxid vor. Auch ist nicht bekannt, in welcher Struktur das Siliziumdioxid am besten am Stoffwechsel teilnimmt. Siliziumdioxid kommt als kristalline Verbindung (z.B. Quarz, Cristobalit), als Glas und als amorphe Substanz vor. Im Kristall und im Glas ist das Siliziumdioxid durch eine nahezu vollständige Vernetzung der SiO_{4/2} Tetraeder bestimmt. Das amorphe Siliziumdioxid mit dem wesentlichen Vertreter Kieselgel hat dagegen ein Netzwerk, das nicht kontinuierlich ist und durch mehr oder weniger innere Oberfläche mit offenen Bindungen (meist SiOH) gekennzeichnet ist.

In Bezug auf eine Degradation von Siliziumdioxid ist die Löslichkeit von Siliziumdioxid in Wasser im Bereich des physiologischen pH-Wertes interessant. Sie liegt für amorphes SiO₂ bei pH 7 bei ca. 150 ppm. Im Kontakt mit lebendem Gewebe erfolgt eine schnellere Lösung als in Pufferlösung pH 7,4. Der Grund hierfür ist unbekannt (Iler, The Chemistry of Silica, 1979 John Wiley &Sons).

Für Wundauflagen wird im Patent US005741509A eine Mischung von Silikonmedium mit "fumed silica" beschrieben. "Fumed silica" besteht aus nichtporösen SiO₂ Partikeln mit einer Dichte von 2.2 g/cm³ und einer Größe zwischen 5 und 50 nm (Wikipedia). Die Dichte, die identisch mit der des Kieselglases ist, zeigt ebenso wie die fehlende Porosität, dass es sich um vollständig vernetzte SiO₂ Strukturen handelt.

Im Patent US2004/0235574A1 wird ebenfall eine Mischung von Silikonmedium mit "fumed silica" beschrieben, wobei zusätzlich antibakteriell wirkende Substanzen hinzugegeben werden.

Das Patent US 7,074,981 B2 beschreibt eine Wundauflage, in der ein Absorbens oder ein Adsorbens in Form von Kieselgel verwendet wird. Ein Absorbens oder ein Adsorbens aus Kieselgel ist nach Stand der Technik ein Xerogel, das im Allgemeinen aus Natriumwasserglaslösung hergestellt wird, wobei eine für ein Xerogel typische Vernetzung der SiO₂ Strukturen erfolgt. (Unter Wikipedia "Adsorption": "Silica gel is a chemically inert, nontoxic, polar and dimensionally stable (< 400 °C or 750 °F) amorphous form of SiO₂. It is prepared by the reaction between sodium silicate and acetic acid, which is followed by a series of after-treatment processes such as aging, pickling, etc. These after treatment methods results in various pore size distributions".)

Mit biologisch degradierbaren Fasern unter anderem aus SiO₂, deren Herstellung und deren Verwendung als Verstärkungsfasern, beschäftigt sich das Patent DE 196 09 551 C1. Hier wird die Herstellung eines spinnbaren Sols beschrieben. Das beschriebene Verfahren und die beschriebenen Anwendung basieren auf der Diplomarbeit von Monika Kursawe von 1995. Die Diplomarbeit basiert wiederum auf den ursprünglichen Synthesevorschriften von Sakka von 1982 (S. Sakka, K. Kamiya; J.Non-Cryst.Solids 48, 1982 31). Sakka beschreibt ein Verfahren, bei dem Gelfäden gesponnen werden, aus denen dann in einem späteren Schritt Glasfasern hergestellt werden. Ausgangsmaterial ist Tetraethylorthosilikat (TEOS), wobei durch Hydrolyse und Kondensation ein spinnfähiges Sol erzeugt wird. Schon Sakka zeigt, dass durch die thixotropen Eigenschaften der Sole nur ein begrenzter Bereich in der Zusammensetzung (TEOS, H₂O, Lösungsmittel (im Allgemeinen Ethanol) und Katalysator) zu spinnbaren Solen führt. Insbesondere das Molverhältnis von Wasser zu TEOS muss um r_{w} 2 liegen.

In der Dissertation "Entwicklung eines Verfahrens zur Herstellung degradierbarer Kieselgelfasern für die Medizintechnik" (Monika Kursawe 1999), einer Weiterentwicklung der Diplomarbeit von 1995 von Kursawe, steht, dass der wichtigste Unterschied zwischen dem in ihrer Diplomarbeit beschriebenen und dem Verfahren von Sakka der ist, dass nach der Kondensation eine Solreifung eingeführt wurde und das als Katalysator Salpetersäure anstatt Salzsäure verwendet wurde. In der Dissertation wird dann das Verfahren so optimiert, dass die Herstellung hochwertiger Kieselgelfasern in größeren Mengen erfolgen kann. Der Prozess basiert auf der leicht modifizierten, in der Diplomarbeit beschriebenen Synthesevorschrift von Sakka.

Das Patent DE 37 80 954 T2 beschreibt ein Verfahren von Siliziumdioxid-Glasfasern, wobei auch hier das Grundverfahren von Sakka modifiziert wurde.

Das Patent DE 10 2007 061 873 A1 beschreibt die Herstellung eines Kieselsolmaterials und die Verwendung als biologisch resorbierbares Material. Als Stand der Technik wird das Patent DE 19609551C1 herangezogen. Als Abgrenzung zu diesem Patent wird aufgeführt, dass hier die Fasern nach dem Spinnen keine optimalen Ergebnisse bei Zytotoxizitätstests erreichen, wobei die Ursachen hierfür vielfältig sein können und nicht mit den wesentlichen Verfahrensschritten zusammenhängen. Auch die zweite Abgrenzung, dass es nach DE 10 2007 061 873 A1 zur Bildung einer "festen Phase" kommt, die ein Filtern des Sols erzwingt, wird nicht mit den wesentlichen Verfahrensschritten in Bezug gesetzt. Der Hauptanspruch 1 des Patentes DE 10 2007 061 873 A1 gibt im Wesentlichen die Herstellanweisung wieder, die M. Kusawe in ihrer Dissertation (1999) beschreibt und die auch in ihrer Diplomarbeit 1995 veröffentlich ist. M. Kusawe teilt die Herstellung des spinnbaren Sols in "Hydrolyse", "Kondensation" und "Reifung" ein. Die "Kondensation" ist nach Kusawe dadurch geprägt, dass dem Sol Ethanol entzogen wird. Das entspricht Anspruch 1 b) des Patentes DE 10 2007 061 873 A1. Die "Reifung" bei Kusawe erfolgt bei ihrem Standardansatz bei 5°C. Das wiederum entspricht dem Anspruch 1 c) und 1 d). Im Beispiel des Patentes DE 10 2007 061 873 A1 wird die Reifung bei 4°C durchgeführt. Auch in den anderen wesentlichen Parametern entspricht das Beispiel dem Standardansatz von Kusawe (z.B. Molverhältnis Wasser/TEOS 1.75, bei Kusawe 1.8). Die Verfahren zur Herstellung spinnbarer Sole der Patente DE 196 09 551 C1 und DE 10 2007 061 873 A1 gehen in ihren wesentlichen Schritten nicht über den Kenntnisstand der Diplomarbeit von Kusawe von 1995 hinaus. Auch die Diplomarbeit basiert stark auf dem Kenntnisstand von Sakka von 1982 (S. Sakka, K.Kamiya; J.Non-Cryst.Solids 48, 1982 31).

Aufgabe der vorliegenden Erfindung ist es, die Struktur von Kieselsäurekondensationsprodukten so zu optimieren, dass eine gesteuerte Degradation bei *in vivo* Anwendung erfolgen kann, und dass diese Kieselsäure-Kondensationsprodukte in bestimmten Applikationsformen wie Granulat, Mikroteilchen, Fasern oder als Schichten auf Implantaten oder Wundauflagen vorliegen können. Hierzu sollen auch Herstellungsverfahren bereitgestellt werden.

Erfindungsgemäß wird diese Aufgabe dadurch gelöst, dass die Kondensation der Kieselsäure in wässriger bzw. in alkoholischer Lösung so gesteuert wird, dass definierte Polyederstrukturen entstehen und dass diese Polyederstrukturen bei den folgenden Verfahrensschritten wie z.B. dem Entfernen des Lösungsmittels erhalten bleiben. Ziel ist, gering vernetzte Kieselsäurestrukturen zu erzeugen, die dadurch gekennzeichnet sind, das sie nicht in einem kontinuierlichen Netzwerk, wie es das Kieselglasnetzwerk ist, eingebaut sind. Der geringste Vernetzungsgrad stellt dabei ein Polyeder aus SiO₂ Tetraedern dar, wobei Fünfer-, Sechs- bzw Siebenerringe eine räumliche Struktur von ca 0.5 nm Durchmesser bilden. Solche Strukturen sind beschrieben in: B. Himmel, Th. Gerber and H. Bürger: WAXS- and SAXS-investigations of structure formation in alcoholic SiO₂ solutions, Journal of Non-Crystalline Solids, Amsterdam, 119(1990)1-13 ;B. Himmel, Th. Gerber and H. Bürger: X-ray diffraction investigations of silica gel structures, Journal of Non-Crystalline Solids, Amsterdam, 91(1987)122-136; B. Himmel, Th. Gerber, W. Heyer and W. Blau: X-ray diffraction analysis of SiO2 structure, Journal of Material Science, Chapman and Hall Ltd., London, 22(1987)1374-1378; Th. Gerber and B. Himmel: The structure of silica glass in dependence on the fictive temperature, Journal of Non-Crystalline Solids, Amsterdam, 92(1987)407-417; Th. Gerber and B. Himmel: The structure of silica glass, Journal of Non-Crystalline Solids, Amsterdam, 83(1986)324-334; B. Himmel, Th. Gerber and H.-G. Neumann: X-ray diffraction investigations of differently prepared amorphous silicas, Physica Status Solidi (a), 88(1985) K127-K130).

Ein Ausgangsmaterial für die Herstellung von Kieselsäurekondensationprodukten ist Tetraethylorthosilikat (TEOS). Mit Wasser entsteht bei Anwesenheit von einem Katalysator Kieselsäure, wobei das Molverhältnis von Wasser/TEOS mindestens 4 sein muss, um zum Startpunkt eine vollständige Hydrolyse zu erreichen. Die entstandene Monokieselsäure kondensiert und bildet Polyederstrukturen von ca. 0.5 nm - 1 nm heraus, sogenannte Primärteilchen, die dann in einer Cluster-Cluster Aggregation fraktale Cluster herausbilden. Diese Cluster wachsen durch den Aggregationsprozess und bei einer bestimmten Größe der Cluster kommt es zur Gelbildung. D.h., die Cluster füllen durch ihre Packung bzw. durch das entstandene Perculationsnetzwerk das Gefäß aus (Th. Gerber, B. Himmel and C. Hübert: WAXS and SAXS investigation of structure formation of gels from sodium water glass, Journal of Non-Crystalline Solids, (1994) vol. 175, p. 160-168 und B. Knoblich, Th. Gerber). C.F. Brinker und G.W. Scherer beschreiben die Gelbildung in einem extra Kapitel in "Sol-gel-science: the physikcs and chemistry of sol-gel processing"(Academic Press; San Diego; 1990). Die Gelbildung ist durch eine extreme Erhöhung der Viskosität charakterisiert.

Auf der Basis von Natriumwasserglaslösung können diese oder analoge Strukturen erzeugt werden. Hierbei werden die Natriumionen bevorzugt mit einem Ionenaustauscher aus der Lösung entfernt. Die verbleibende Kieselsäure liegt hier dann schon als Kondensationsprodukt vor. Es handelt sich um Polyederstrukturen von ca. 0.5 nm Größe, wiederum Primärteilchen genannt, die dann in Anhängigkeit vom pH-Wert durch Aggregation fraktale Cluster bilden, die wiederum zur Gelbildung führen (B. Knoblich, Th. Gerber: Aggregation in SiO2 sols from sodium silicate solutions, Journal of Non-Crystalline Solids 283 (2001) 109-113).

Die Aggregationscluster (Feststoffgerüst, Metalloxid) des Alkogels (Lösungsmittel, Alkohol) bzw. des Hydrogels (Lösungsmittel, Wasser) werden beim Trocknen aufgrund der wirkenden Kapillarkräfte und der ablaufenden Kondensation der inneren Oberfläche (2Si*_{surface}*-OH → Si*_{bulk}*-O-M*_{bulk}* + H₂O) zerstört. Es entsteht ein Xerogel, dessen innere Oberfläche z.B. in dem Fall SiO₂ im Bereich von 25-700 m²/g und dessen Dichte im Bereich über 1.0 g/cm³ liegt. Die definierten Polyederstrukturen in den Primärteilchen vernetzen sich beim Trocknen. Es entsteht ein kontinuierliches Netzwerk mit der oben beschriebenen hohen inneren Oberfläche. Die Vernetzung des Siliziumdioxides wird erhöht.

Bei der Herstellung von Aerogelen wird dieser Prozess verhindert. Hierfür gibt es nach dem Stand der Technik zwei grundlegend verschiedene Wege.

Zum einen werden überkritische Trocknungsverfahren angewandt. Hierdurch wird die Wirkung der Kapillarkräfte verhindert, da der Phasenübergang Flüssig/Gas durch ein entsprechendes Temperatur-Druck-Regime umgangen wird. Als Lösungsmittel werden hierbei Alkohole (Methanol, Ethanol, Propanol) oder flüssiges CO₂ verwendet, die durch Austauschverfahren das ursprüngliche Lösungsmittel, meist H₂O, ersetzen müssen (S.S. Kistler, Phys. Chem. 36 (1932) 52-64, EP 171722; DE 1811353; US 3672833; DE 39 24 244 A1; PCT/EP94/02822). Durch die verwendeten Autoklaven sind die Verfahren sehr kostenaufwändig.

Andererseits existieren nach dem Stand der Technik Verfahren, die eine unterkritische Trocknung von Aerogelen erlauben. Kernpunkt des Verfahrens nach PCT/US94/05105 ist eine Modifikation des Kontaktwinkels zwischen Lösungsmittel und Feststoffgerüst. Hierdurch wird der Kapillardruck verringert und die Struktur des feuchten Gels nahezu erhalten. Der Kontaktwinkel wird durch eine Modifikation der inneren Oberfläche des Feststoffgerüstes im feuchten Gel erreicht. Hierzu erfolgt eine Reaktion der inneren Oberfläche mit RₓSiX_{y}. R ist eine organische Gruppe und X ist ein Halogen. Bei diesem Verfahren ist ein mehrfacher Lösungsmittelaustausch notwendig. Im Patent DE 19538333 A1 wird eine Modifikation der inneren Oberfläche des feuchten Gels mit Si*_{surface}*O-Z realisiert, wobei Z eine beliebige Gruppe ist, die die Kondensation der inneren Oberfläche beim Trocknen verhindern soll.

Die zur Herstellung von Aerogelen verwendeten Verfahren werden im Rahmen der vorliegenden Erfindung nicht genutzt, insbesondere kommt es nicht zu einer Gelbildung.

EP 0 881 192 A1 offenbart ein Verfahren zur Herstellung von Kieselsäurestrukturen, bei dem eine Stabilisierung des Sols durch Bildung von Si-C-Bindungen erfolgt. Eine Veresterung der Kieselsäurestrukturen wird nicht beschrieben. WO 01/52618 A2 offenbart ein Verfahren zur Herstellung von Kieselsäurestrukturen, die als Füllstoff bei der Gummiherstellung dienen. Eine Veresterung der Kieselsäurestrukturen wird nicht beschrieben. WO 2009/030919 A2 beschreibt ein Biomaterial aus Kieselsäurestrukturen, die in einem Sol durch Hydrolyse von Silanen hergestellt werden. WO 2005/087284 A2 beschreibt ein Knochenersatzmaterial aus Kieselsäurestrukturen einer Größe von 10 nm bis 10 µm. Kieselsäurestrukturen mit Si-OR-Gruppen an deren Oberfläche werden jedoch weder in der WO 2009/030919 A2 noch in der WO 2005/087284 A2 beschrieben.

Die Aufgabe der vorliegenden Erfindung ist es, Materialien mit einer definierten Vernetzung der Kieselsäure herzustellen. Daraus können Produkte wie Mikroteilchen, Fasern oder Schichten hergestellt werden.

Erfindungsgemäß wird diese Aufgabe dadurch gelöst, dass eine weitere Kondensation verhindert wird, wenn bestimmte Vernetzungen der Kieselsäure erreicht werden, insbesondere wenn die gewünschte Größe der Kieselgelcluster durch Kondensation erreicht wurde, wobei die gewünschte Größe der Kieselgelcluster bevorzugt im Bereich von ca. 0.5 nm bis hin zu ca. 1000 nm, mehr bevorzugt von 0.5 nm bis 20 nm, mehr bevorzugt bis 10 nm, bis 5 nm, bis 4v nm, bis 3 nm, bis 2 nm oder bis 1 nm.

Erfindungsgemäß wird diese Aufgabe in einer Ausführungsform dadurch gelöst, dass eine weitere Kondensation verhindert wird, wenn bestimmte Vernetzungen der Kieselsäure erreicht werden. Im Gegensatz zur Aerogelherstellung ist das noch vor der Gelbildung.

Eine sehr geringe Vernetzung haben die oben beschriebenen Polyeder in den Primärteilchen der Polykieselsäure oder kleinen (s.o.) Aggregaten derselben, die im Rahmen dieser Erfindung ebenfalls als Primärteilchen bezeichnet werden können. Diese werden konserviert, indem nach ihrer Entstehung das vorhandene Wasser durch ein organisches Lösungsmittel ersetzt wird. Das Lösungsmittel kann insbesondere einen Alkohol mit 1-10 C-Atomen sein, z.B. Methanol, Ethanol, Propanol, Butanol, Pentanol, Hexanol, etc. oder Phenol als Arylgruppe, ggf. in einem Grad substituiert, der mit der erfindungsgemäßen Verwendung verträglich ist, insbesondere ist dieses Lösungsmittel Ethanol oder Phenol, bevorzugt Ethanol. In den an der Oberfläche der Primärteilchen vorhandenen Si*_{Primärteilchen}*-OH Gruppen wird das Proton durch eine organische Gruppe ersetzt, wie eine Alkyl oder Arylgruppe, bevorzugt mit 1-10 C-Atomen, z.B. Methyl, Ethyl, Propyl, Butyl, Pentyl, Hexyl, etc. oder Phenyl als Arylgruppe, ggf. in einem Grad substituiert, der mit der erfindungsgemäßen Verwendung verträglich ist, insbesondere ist diese Gruppe Ethyl oder Phenyl, bevorzugt Ethyl. Es wird also eine Veresterung durchgeführt. Die Hydrolyse/Veresterung ist eine Gleichgewichtsreaktion, die in Richtung Veresterung verschoben wird. Das kann z.B. geschehen, indem bei Anwesenheit eines Katalysators das Wasser entzogen wird. Das Wasser kann beseitigt werden, indem das Wasser-Lösungsmittel (z.B. Ethanol) Gemisch abdestilliert und durch ein Molekularsieb das Wasser im Destillat entzogen und das Lösungsmittel (z.B. Ethanol) zurückgeführt wird.

Der Veresterungsgrad in den erfindungsgemäß erhaltenen Materialien liegt bevorzugt bei ca. 50% bis ca. 95%, mehr bevorzugt bei ca. 60% bis ca. 80% oder bei ca. 70% bis ca. 75%.

Bevorzugt liegt die Größe der Kieselgelcluster/-Aggregate, welche durch Kondensation erreicht wurde, im Bereich von ca. 0,5 nm bis hin zu 1000 nm liegt, insbesondere ca. 0,5 nm bis ca. 20 nm oder bis ca. 10 nm, bis ca. 4 nm, bis ca. 2 nm oder bis ca. 1 nm. Eine Gelbildung wird damit durch das erfindungsgemäße Verfahren verhindert.

Die veresterten Primärteilchen (bzw. die Aggregate derselben) in alkoholischer Lösung ohne Wasser sind nun stabil. Sie können weiterverarbeitet werden. Werden sie nun z.B. sprühgetrocknet, so entweicht der Alkohol aus den Tröpfchen und es entsteht in den Mikroteilchen (ursprüngliche Tröpfchen) eine dichte Packung der Primärteilchen ohne eine weitere Vernetzung.

Sollen aus den veresterten Primärteilchen sekundäre Strukturen wie Mikropartikel oder Fasern erzeugt werden, sollte der Veresterungsgrad zwischen ca. 60% und 95% liegen, damit eine Verbindung der Primärteichen über Silanolbrücken erfolgen kann, eine Vernetzung der SiO₂ Struktur aber verhindert wird.

Für eine medizinische Anwendung können die organischen Gruppen wie Ethylgruppen anschließend durch eine Hydrolyse oder durch eine Behandlung im Sauerstoffplasma wieder entfernt werden. Benachbarte SiOH Gruppen können dann auch kondensieren. Es entsteht aber keine kontinuierlich vernetzte Struktur wie beim Xerogel.

Mit Hilfe von Small Angle X-ray Scattering (SAXS) und Wide Small Angle X-ray Scattering (WAXS) kann die Vernetzung der SiO₂-Strukturen dokumentiert werden (B. Himmel, Th. Gerber and H. Bürger: WAXS- and SAXS-investigations of structure formation in alcoholic SiO2 solutions, Journal of Non-Crystalline Solids, Amsterdam, 119(1990)1-13; B. Himmel, Th. Gerber and H. Bürger: X-ray diffraction investigations of silica gel structures, Journal of Non-Crystalline Solids, Amsterdam, 91(1987)122-136; B. Himmel, Th. Gerber, W. Heyer and W. Blau: X-ray diffraction analysis of SiO2 structure, Journal of Material Science, Chapman and Hall Ltd., London, 22(1987)1374-1378; Th. Gerber and B. Himmel: The structure of silica glass in dependence on the fictive temperature, Journal of Non-Crystalline Solids, Amsterdam, 92(1987)407-417; Th. Gerber and B. Himmel: The structure of silica glass, Journal of Non-Crystalline Solids, Amsterdam, 83(1986)324-334; B. Himmel, Th. Gerber and H.-G. Neumann: X-ray diffraction investigations of differently prepared amorphous silicas, Physica Status Solidi (a), 88(1985) K127-K130).

Eine wesentliche Erkenntnis, die zur Kontrolle der Herstellungsprozesse genutzt werden kann, ist es, dass die Position des Hauptmaximums der WAXS Streukurve von unterschiedlichen SiO₂ Strukturen ein Maß des Vernetzungsgrades ist. Die geringste Vernetzung (eines vollständigen Polyeders) hat ein Maximum bei ca. 16.4 nm⁻¹. Eine vollständige Vernetzung, wie sie im Kieselglas vorliegt, hat ein Maximum bei ca. 14.7 nm⁻¹. In den Beispielen wird das genutzt.

Bevorzugt liegt ein Vernetzungsgrad des erfindungsgemäßen Materials vor, die zu einem Hauptmaximum einer WAXS Streukurve von mehr als 14.7 nm⁻¹ und weniger als 16.4 nm⁻¹ führt. Das Hauptmaximum liegt vorzugsweise zwischen 15.5 nm⁻¹ und weniger als 16.4 nm⁻¹, z.B. bei etwa 16.0 nm⁻¹.

Aus den veresterten Primärteilchen (bzw. Aggregaten derselben) kann nun eine spinnfähige Lösung hergestellt werden. Hierzu wird soviel Alkohol entfernt, dass die Viskosität im Bereich von ca. 0.6-0.8 Pas, bevorzugt von ca. 0.7 Pas liegt. Die Lösung wird nun mit einem Druck von ca. 9-11 bar, bevorzugt von ca. 10 bar durch Düsen gedrückt. In einem Spinnturm trocknen die Fäden im temperierten Luftstrom und werden auf einem Gitter aufgefangen. Ähnlich wie in den Mikroteilchen liegen die veresterten Primärteilchen als dichte Packung in den Fäden vor. Auch hier können die Ethylgruppen wieder entfernt werden.

Die veresterten Primärteilchen (bzw. Aggregate derselben) in alkoholischer Lösung können auch zur Herstellung von Schichten auf Implantaten oder Wundauflagen genutzt werden. Hierzu können bekannte Beschichtungsverfahren wie *dip coating, spin coating* oder *spray coating* genutzt werden. Entscheidend ist nun wieder, dass die Schicht durch eine Packung der Primärteilchen aufgebaut wird, wobei beim Trocknen kein kontinuierliches Netzwerk wie in einem Xerogel entsteht.

Bevorzugt wird die Veresterung durch einen Katalysator erleichtert, insbesondere eine Säure oder Base oder ein Ionenaustauscher. Bevorzugt wird eine organische Säure (z.B. Ameisensäure, Essigsäure, Äpfelsäure, Oxalsäure) eingesetzt, aber auch anorganische Säuren wie HCl können verwendet werden. Bevorzugt ist der Katalysator in den im Produkt enthaltenen Mengen gewebeverträglich. Orientierung bei der Auswahl eines Katalysators bietet auch C.F. Brinker und G.W. Scherer in "Sol-gel-science: the physikcs and chemistry of sol-gel processing", Kapitel 2.3.1. Effects of Catalysts (Academic Press; San Diego; 1990).

Bei den bisher beschriebenen Prozessschritten ist der Katalysator am Ende des Verfahrens noch im entstandenen Biomaterial, was gegebenenfalls die biologische Wirksamkeit beeinflussen könnte. Der Katalysator kann aber auch entfernt werden, so dass in dem erhaltenen Material keine oder keine signifikanten Mengen an Katalysator mehr vorliegen. Zum einen kann das verhindert werden, indem organische Säuren als Katalysator sowohl für die Hydrolyse als auch für die Veresterung verwendet werden. Da Essigsäure und Ameisensäure selbst leicht verestern, wird hier Oxalsäure bevorzugt. Durch eine Sauerstoffplasmabehandlung kann die Säure leicht entfernt werden. Zum anderen kann z.B. ein Ionenaustauscher z.B. sulfoniertes Polystyrol als Katalysator verwendet werden. Der Vorteil ist, dass der Ionenaustauscher (Polystyrolkügelchen) leicht aus der fertigen Lösung entfernt werden kann.

Der beschriebene Prozess kann auch durchgeführt werden, wenn die Aggregation der Primärteilchen begonnen hat. Sind einige Primärteilchen durch eine Kondensationsreaktion verbunden und werden dann die Veresterung und die oben beschriebenen Trocknungsverfahren durchgeführt, kommt es zur dichten Packung dieser Aggregate, die nicht weiter vernetzen.

Der Vernetzungsgrad der Kieselsäure kann in wässriger Lösung vor der Veresterung kontrolliert erhöht werden. Das Sol mit den Primärteilchen wird auf einen pH-Wert zwischen 7 und 9 eingestellt, bevorzugt ca. pH 7, bis ca. 7,4, ca. pH 8 oder ca. pH 9. Da der isoelektrische Punkt der Kieselsäure bei ca. pH 2 liegt, sind die Teilchen stark negativ geladen und können daher nicht aggregieren. Es kommt jedoch zu einem Wachsen der Nanoteilchen durch Ostwaldreifung. Da in den Nanoteilchen die Kieselsäure ein Netzwerk bildet, wird der Vernetzungsgrad mit dem Teilchenwachstum größer. Durch Austausch des Lösungsmittels Wasser und durch eine Veresterung wird der Prozess gestoppt und konserviert.

Anschließend können die verschiedenen Darreichungsformen (Granulat, Mikroteilchen, Fäden, Schicht) mit den nun größeren, in sich mehr vernetzten Primärteilchen, hergestellt werden.

Bei der Verwendung der beschriebenen gering vernetzten Kieselsäurestrukturen zur Verbesserung der Wundheilung ist es von Vorteil, ein geeignetes Trägermaterial zu nutzen. Hierbei bieten sich Wundauflagen nach dem Stand der Technik an. Bevorzugt sollten resorbierbare Materialien verwendet werden, da mit der Resorption des Trägers das SiO₂ freigesetzt wird.

Die Erfindung stellt ein Verfahren zu Verfügung, um gering vernetzte Kieselsäurestrukturen zu erzeugen, wobei:
a) ein Sol erzeugt wird.
b) das im Sol enthaltene Wasser durch ein wasserlösliches, organisches Lösungsmittel ersetzt wird.
c) im Sol nach Beseitigung des Wassers durch ein organisches Lösungsmittel die im Sol an der inneren Oberfläche der Kieselsäurecluster vorhandenen Si*_{surface}*OH-Gruppen zu Si*_{surface}*OR verestert, wobei R ein organischer Rest ist, wobei R eine Alkyl- oder Arylgruppe ist, die ausgewählt ist aus der Gruppe bestehend aus Methyl, Ethyl, Propyl, Butyl, Pentyl, Hexyl, und Phenyl, und wobei der Veresterungsgrad der Kieselsäurecluster größer als 50% ist.

In dem Verfahren kann das Sol durch Hydrolyse von Tetraethylorthosilicat (TEOS) erzeugt werden, wobei bevorzugt ein r_{W} Wert (Molverhältnis von Wasser zu TEOS) von 4 genutzt wird. Als Lösungsmittel wird bevorzugt Ethanol verwendet. Als Katalysator für die Hydrolyse wird bevorzugt eine organische Säure, bevorzugt Oxalsäure, genutzt. Das Wasser kann dann durch ein organisches Lösungsmittel, bevorzugt Ethanol, ersetzt werden, wenn die gewünschte Größe der Kieselgelcluster durch Kondensation erreicht wurde, wobei die Größe im bevorzugten Bereich von ca. 0.5 nm bis hin zu 1000 nm liegt, insbesondere ca. 0,5 nm bis ca. 20 nm oder 1 bis 10 nm. Das Sol kann auch durch Ionenaustausch von Natriumwasserglaslösung erzeugt werden. wobei das Wasser dann durch ein organisches Lösungsmittel, bevorzugt Ethanol, ersetzt wird, wenn die gewünschte Größe der Kieselgelcluster durch Kondensation erreicht wurde, wobei die bevorzugte Größe im Bereich von ca. 0,5 nm bis hin zu 1000 nm liegt, insbesondere ca. 1 nm bis ca. 10 nm oder 2 bis 5 nm.

Die an der inneren Oberfläche der Kieselsäurecluster vorhandenen-Gruppen werden zu Si*_{surface}*OR verestert, wobei R eine organische Gruppe ist, wie eine Alkyl oder Arylgruppe, bevorzugt mit 1-10 C-Atomen, z.B. Methyl, Ethyl, Propyl, Butyl, Pentyl, Hexyl, etc. oder Phenyl als Arylgruppe, ggf. in einem Grad, der mit der erfindungsgemäßen Verwendung verträglich ist, ersetzt, insbesondere ist diese Gruppe Ethyl oder Phenyl, bevorzugt Ethyl. Die Veresterung wird dabei bevorzugt dadurch realisiert, dass bei Anwesenheit einer Säure, bevorzugt einer organischen Säure, besonders bevorzugt Oxalsäure, das Wasser, das bei der Veresterung entsteht, entfernt wird. Dabei wird ein Veresterungsgrad von größer als 50% oder erreicht, insbesondere 60% oder größer oder 70% oder größer.

Bevorzugt wird in den erfindungsgemäßen Verfahren ein Sprühtrocknungsverfahren verwendet.

Bevorzugt wird in den erfindungsgemäßen Verfahren das im Sol enthaltene Wasser durch ein organisches Lösungsmittel ersetzt, wenn die Kieselgelcluster eine Größe von 0.5 bis 4 nm haben. Dabei ist bevorzugt das organische Lösungsmittel Ethanol. Es wird eine Veresterung durchgeführt und dass das veresterte Sol bevorzugt soweit eingekocht, dass der Feststoffgehalt (SiO₂) im Sol bevorzugt im Bereich von 3 bis 25 Gew. % liegt. Bevorzugt wird dieses Sol sprühgetrocket, wobei die Sprühparameter bevorzugt so gewählt werden, dass hohle Mikropartikel entstehen.

Bevorzugt wird in den erfindungsgemäßen Verfahren das Lösungsmittel soweit entfernt, dass die Viskosität des Sols im Bereich von 0.1 Pas bis 10 Pas, bevorzugt im Bereich von 0.3 Pas bis 0.7 Pas liegt. Dieses Sol kann in bekannten Verfahren zu Fäden gesponnen werden.

Bevorzugt wird in den erfindungsgemäßen Verfahren das im Sol enthaltene Wasser durch ein organisches Lösungsmittel ersetzt, wenn die Kieselgelcluster eine Größe von 0.5 bis 4 nm haben. Dabei ist bevorzugt das organische Lösungsmittel Ethanol. Es wird eine Veresterung durchgeführt und dass das veresterte Sol bevorzugt soweit eingekocht, dass das Sol eine Viskosität bevorzugt im Bereich von 0.3 Pas bis 0.7 Pas hat. Bevorzugt wird dieses Sol in bekannten Verfahren zu Gelfäden gesponnen.

Bevorzugt wird in den erfindungsgemäßen Verfahren durch Entfernen oder Hinzufügen von Lösungsmittel ein Feststoffgehalt (SiO₂) im Sol erhalten wird, der im Bereich von 3 bis 25 Gew.% liegt. Bevorzugt wird dieses Sol für bekannte Beschichtungsverfahren wie *dip coating, spin coating* oder *spray coating* genutzt.

Bevorzugt wird in den erfindungsgemäßen Verfahren ein Katalysator verwendet, insbesondere eine organische Säure, bevorzugt Oxalsäure. Alternativ kann der verwendete Katalysator ein saurer Ionenaustauscher, bevorzugt sulfoniertes Polystyrol R-SO₃H, sein.

Bevorzugt wird in den erfindungsgemäßen Verfahren das Sol einem Reifungsprozess unterzogen, bevor die Oberfläche, wie oben beschrieben, konserviert wird, wobei das Lösungsmittel Wasser oder ein Gemisch aus Wasser und organischem Lösungsmittel ist und ein pH-Wert >7 eingestellt wird.

Bevorzugt werden in den erfindungsgemäßen Verfahren die organischen Bestandteile oxidiert, wobei bevorzugt ein Sauerstoffplasma genutzt wird. Dies findet insbesondere nach der Veresterung statt.

In einer Ausführungsform werden in den erfindungsgemäßen Verfahren die hergestellten, gering vernetzten Kieselsäurestrukturen (bevorzugt nach der Veresterung) in eine wässrige Lösung von Polymeren gegeben werden, wobei der Polymeranteil in der Lösung bevorzugt zwischen 2 und 15 Gew.% liegt und der Anteil des SiO₂ in Bezug zur Polymer/Wasserlösung bevorzugt im Bereich von 2 bis 40 Gew.% liegt, die Lösung mit den homogen vermischten Kieselsäurestrukturen in eine Form gegossen und gefriergetrocknet wird. Dabei kann das wasserlösliche Polymer Polyvinylpyrrolidon sein. Anstatt des wasserlöslichen Polymers kann auch Kollagen verwendet werden.

Gegenstand der Erfindung ist auch ein Biomaterial, erhältlich nach diesem Verfahren, welches aus SiO₂ Polyederstrukturen aufgebaut ist, die eine Größe von 0.5 nm bis 4 nm aufweisen, und die an der Oberfläche Si-OH-, Si-OR- oder SiR-Gruppen aufweisen, wobei R eine organische Gruppe ist, wie eine Alkyl oder Arylgruppe, bevorzugt mit 1-10 C-Atomen, z.B. Methyl, Ethyl, Propyl, Butyl, Pentyl, Hexyl, etc. oder Phenyl als Arylgruppe, ggf. in einem Grad substituiert, der mit der erfindungsgemäßen Verwendung verträglich ist, insbesondere ist diese Gruppe Methyl, Ethyl oder Phenyl, bevorzugt Ethyl. Die Größe dieser Strukturen ist stabil.

Das Material kann als Granulat vorliegen. Das Material kann auch als Mikroteilchen, bevorzugt hohles Mikroteilchen und/oder bevorzugt mit einem Durchmesser im Bereich von 2 µm bis 100 µm vorliegen. Das Material kann auch als Fäden, bevorzugt mit einem Durchmesser im Bereich von 1 µm bis 50 µm, vorliegen. Das Material kann auch als Schicht auf Implantaten oder Wundauflagen vorliegen.

Das erfindungsgemäße Material kann in ein Trägermaterial eingebettet sein, wobei bevorzugt eine organische Membran, ein organisches Vlies oder ein organischer Schwamm verwendet wird, wobei bevorzugt ein biodegradierbares organisches Material zur Anwendung kommt. Insbesondere kann als Träger ein Gelatineschwamm oder ein Schwamm aus Polyvinylpyrrolidon verwendet werden.

Gegenstand der Erfindung ist auch eine Verwendung des erfindungsgemäßen Materials (Biomaterials) für Medizinprodukte, insbesondere für solche, die eine stützende oder abschirmende Funktion haben und/oder gleichzeitig durch die Degradation als Lieferant für das die Geweberegeneration unterstützende Siliziumdioxid dienen können. Im Rahmen der Erfindung wird die Bezeichnung Medizinprodukt als austauschbar zu medizinische oder pharmazeutische Zusammensetzung oder Medikament verwendet, da die Klassifizierung von nationalem Recht abhängt, die Substanz der Erfindung jedoch nicht ändert.

Gegenstand der Erfindung ist auch eine Verwendung des erfindungsgemäßen Materials (Biomaterials) als Nahrungsergänzungsmittel.

### Legenden

- Fig. 1: zeigt eine fotografische Abbildung des Vlies aus Silicafäden nach Beispiel 1.
- Fig. 2: zeigt eine rasterelektronenmikroskopische Abbildung eines Gelfadens nach Beispiel 1.
- Fig. 3: dokumentiert eine Röntgenbeugungsuntersuchung der Silicagelfäden nach Beispiel 1 nach unterschiedlichen Temperaturbehandlungen bzw. unbehandelt (untreated).
- Fig. 4: zeigt eine rasterelektronenmikroskopische Aufnahme der im Beispiel 2 hergestellten Mikropartikel
- Fig. 5: zeigt die Röntgenbeugung der in Beispiel 2 bis 5 hergestellten Mikropartikel mit einem Veresterungsgrad von ca. 50% (untere Kurve), ca. 75% (mittlere Kurve) und ca. 90% (obere Kurve).
- Fig. 6: zeigt rasterelektronenmikroskopische Abbildungen eines Polyvinylpyrrolidonschwamms mit eingebundenen Mikropartikels in verschiedenen Vergrößerungen. A: Skala=200 µm, B: Skala= 40 µm.
- Fig. 7: zeigt rasterelektronenmikroskopische Abbildungen eines Gelatineschwamms mit eingebundenen Mikropartikels in verschiedenen Vergrößerungen. A: Skala=40 µm, B: Skala= 9 µm.

### Beispiele

### Beispiel 1

100 g TEOS, 35 g H₂0, 3 g Oxalsäure und 800 g Ethanol werden in einem Gefäß 20 Minuten mit einem Magnetrührer vermischt. Während dieser Zeit findet die Hydrolyse des TEOS statt. Anschließend wird über eine azeotrope Destillation das Wasser entfernt, wobei Ethanol, nachdem über ein Molekularsieb das Wasser entzogen wurde, in das Gefäß zurückgeführt wird. Der Prozess wird 2 Stunden durchgeführt.

Danach wird die Rückführung des Ethanols unterbunden und soweit Lösungsmittel durch Destillation entfernt, bis die *in situ* ermittelte Viskosität im Bereich zwischen 0.5 und 0.7 Pas ist.

Das so veresterte Sol wird mit 11 bar durch ein Düsensystem mit 7 Düsen gedrückt, wobei die Düsen einen Durchmesser von 0.2 mm haben.

Das Spinnen erfolgt in einem 3 m hohen Turm (Edelstahlrohr von 30 cm Durchmesser) in einem Strom von Luft von einer Temperatur von 60°C. Durch einen verwirbelten Luftstrom quer zur Spinnrichtung mit einer Temperatur von 150°C werden die Gelfäden auf einem Sieb aufgefangen, so dass ein vliesartiges Gewebe entsteht.

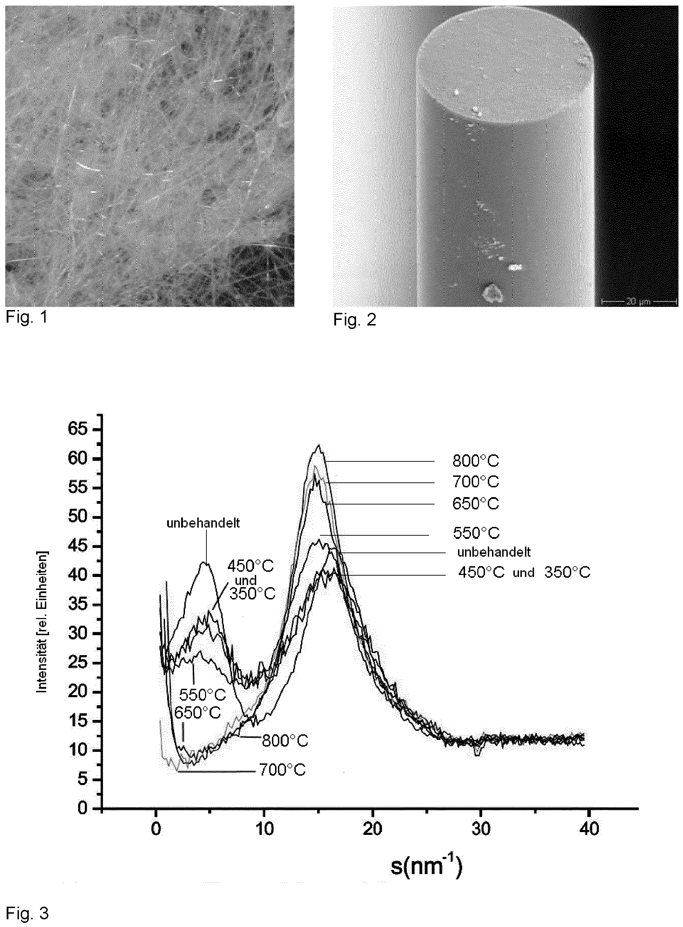

Fig. 1 zeigt eine fotografische Abbildung des Gelvlies. In der rasterelektronenmikroskopischen Abbildung (Fig. 2) erscheint das Innere der Faser homogen. Die Röntgenbeugungsuntersuchungen (Fig. 3) dokumentiert jedoch, dass die Faser durch eine Packung von ca. 0.5 bis 1nm großen Teilchen bestimmt ist. Das zeigt der Peak bei ca 5 nm⁻¹. Der Peak bei 16.4 nm⁻¹ der unhehandelten Probe (Hauptmaximums der WAXS Streukurve) dokumentiert eine geringe Vernetzung der SiO₂ Polyederstrukturen. Erst die Temperaturbehandlung führt zur besseren Vernetzung, bis ab ca. 650°C ein für Kieselglas typisches vollständiges Netzwerk entstanden ist. Der Peak bei ca. 5 nm⁻¹ ist dann verschwunden.

Das Beispiel dokumentiert, dass nach dem beschriebenen Verfahren Gelfäden entstanden sind, die SiO₂ Polyederstrukturen mit dem geringst möglichen Vernetzungsgrad enthalten.

### Beispiel 2

100 g TEOS, 35 g H₂0, 3 g Oxalsäure und 800 g Ethanol werden in einem Gefäß 20 Minuten mit einem Magnetrührer vermischt. Während dieser Zeit findet die Hydrolyse des TEOS statt. Anschließend wird über eine azeotrope Destillation das Wasser entfernt, wobei Ethanol, nachdem über ein Molekularsieb das Wasser entzogen wurde, in das Gefäß zurückgeführt wird. Der Prozess wird 1 Stunde durchgeführt.

Danach wird die Rückführung des Ethanols unterbunden und soweit Lösungsmittel durch Destillation entfernt, bis 220 g des Ansatzes übrigbleiben (Der SiO₂ Anteil beträgt hierbei ca. 28 g).

Es erfolgt ein Sprühtrocknen mit einem Büchli 290 mit Inert Loop.

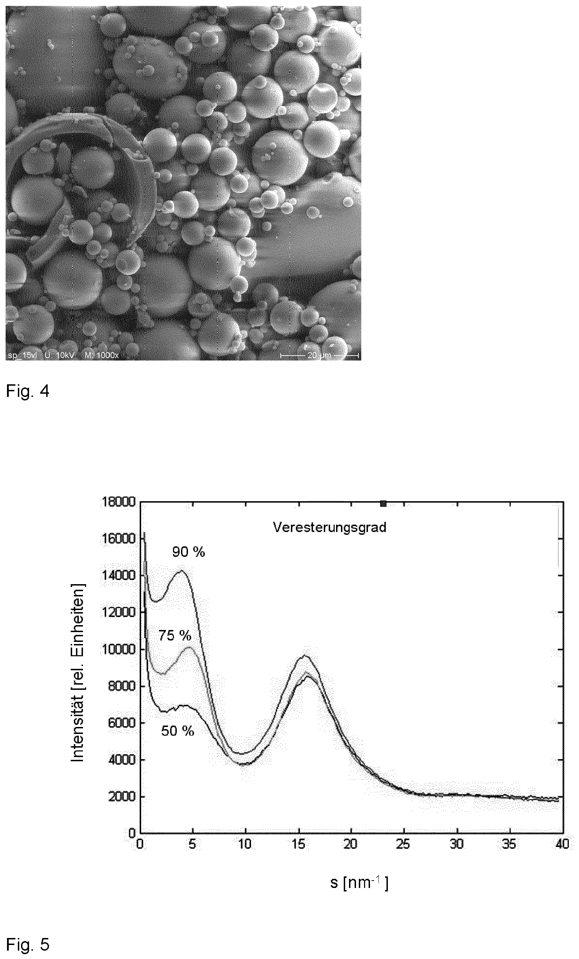

Fig. 4 zeigt die entstandenen Mikroteilchen im Rasterelektronenmikroskop. Die Röntgenbeugung in der Fig. 5 (Kurve mit Veresterungsgrad von ca. 50%, bestimmt mit IR-Spektroskopie) zeigt den typischen Peak bei ca 5 nm⁻¹.

### Beispiel 3

100 g TEOS, 35 g H₂0, 3 g Oxalsäure und 800 g Ethanol werden in einem Gefäß 20 Minuten mit einem Magnetrührer vermischt. Während dieser Zeit findet die Hydrolyse des TEOS statt. Anschließend wird über eine azeotrope Destillation das Wasser entfernt, wobei Ethanol, nachdem über ein Molekularsieb das Wasser entzogen wurde, in das Gefäß zurückgeführt wird. Der Prozess wird 2 Stunden durchgeführt.

Danach wird die Rückführung des Ethanols unterbunden und soweit Lösungsmittel durch Destillation entfernt, bis 220 g des Ansatzes übrigbleiben (Der SiO₂ Anteil beträgt hierbei ca. 28 g).

Es erfolgt ein Sprühtrocknen mit einem Büchli 290 mit Inert Loop.

Die Röntgenbeugung der entstandenen Mikroteilchen ist in Fig. 5 (Kurve mit Veresterungsgrad von ca. 75%, bestimmt mit IR-Spektroskopie) dargestellt.

### Beispiel 4

100g TEOS, 35 g H₂0, 3 g Oxalsäure und 800 g Ethanol werden in einem Gefäß 20 Minuten mit einem Magnetrührer vermischt. Während dieser Zeit findet die Hydrolyse des TEOS statt. Anschließend wird über eine azeotrope Destillation das Wasser entfernt, wobei Ethanol, nachdem über ein Molekularsieb das Wasser entzogen wurde, in das Gefäß zurückgeführt wird. Der Prozess wird 4 Stunden durchgeführt.

Danach wird die Rückführung des Ethanols unterbunden und soweit Lösungsmittel durch Destillation entfernt bis 220 g des Ansatzes übrigbleiben (Der SiO₂ Anteil beträgt hierbei ca. 28 g).

Es erfolgt ein Sprühtrocknen mit einem Büchli 290 mit Inert Loop.

Die Röntgenbeugung der entstandenen Mikroteilchen ist in Fig. 5 (Kurve mit Veresterungsgrad von ca. 90%, bestimmt mit IR-Spektroskopie) dargestellt.

### Beispiel 5

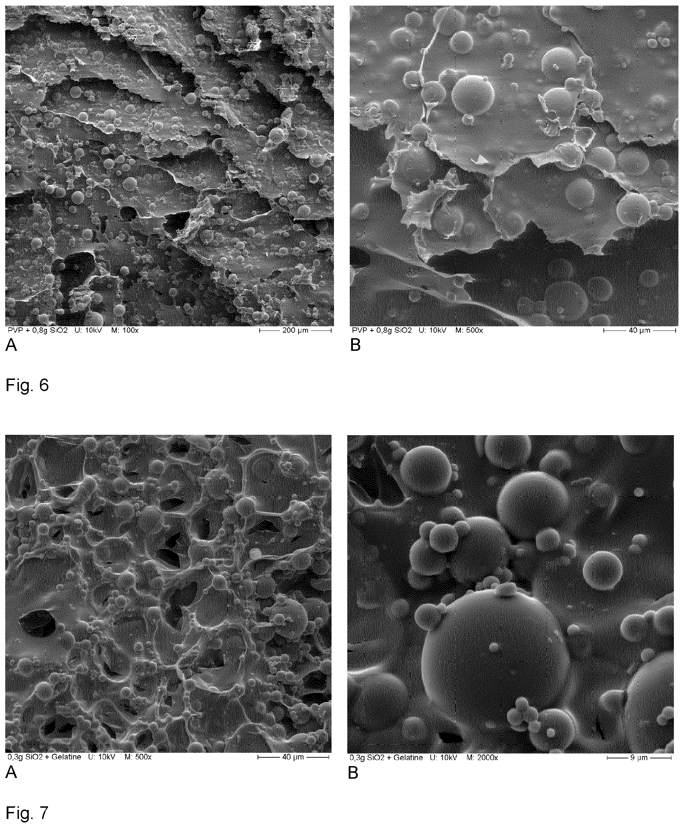

In 100 g H₂O werden 7 g Polyvinylpyrrolidon gelöst. Die im Beispiel 4 hergestellten Mikropartikel werden für eine Stunde im Sauerstoffplasma behandelt, wodurch die Ethylgruppen und Reste des Katalysators (Oxalsäure) entfernt werden. Anschließend werden 3 g dieser Mikropartikel in die Lösung gegeben und homogen verteilt. Anschließend wird das Material in Schalen gegeben, sodass die Flüssigkeitshöhe 5 mm beträgt. Das Gemisch aus H₂O, Polyvinylpyrrolidon und den Mikroteilchen wird gefroren und gefriergetrocknet. Es entsteht ein Polyvinylpyrrolidonschwamm mit eingebundenen Mikropartikeln, wie es in der rasterelektronenmikroskopischen Abbildung Fig. 6A und B zu erkennen ist.

### Beispiel 6

In 100 g H₂O werden 7 g Gelatine gelöst. Die im Beispiel 4 hergestellten Mikropartikel werden für eine Stunde im Sauerstoffplasma behandelt, wodurch die Ethylgruppen und Reste des Katalysators (Oxalsäure) entfernt werden. Anschließend werden 3 g dieser Mikropartikel in die Lösung gegeben und homogen verteilt. Anschließend wird das Material in Schalen gegeben, sodass die Flüssigkeitshöhe 5 mm beträgt. Das Gemisch aus H₂O, Gelatine und den Mikroteilchen wird gefroren und gefriergetrocknet. Es entsteht ein Gelatineschwamm mit eingebundenen Mikropartikeln, wie es in der rasterelektronenmikroskopischen Abbildung Fig. 7A und B zu erkennen ist.

## Patentansprüche

1. Ein Verfahren zur Herstellung gering vernetzter Kieselsäurestrukturen, bei dem man:
a) ein Sol erzeugt,
b) das im Sol enthaltene Wasser durch ein wasserlösliches, organisches Lösungsmittel ersetzt,
c) nach Beseitigung des Wassers durch ein organisches Lösungsmittel die im Sol an der inneren Oberfläche der Kieselsäurecluster vorhandenen Si*_{surface}*OH-Gruppen zu Si*_{surface}*OR verestert, wobei R ein organischer Rest ist, wobei R eine Alkyl- oder Arylgruppe ist, die ausgewählt ist aus der Gruppe bestehend aus Methyl, Ethyl, Propyl, Butyl, Pentyl, Hexyl, und Phenyl, und wobei der Veresterungsgrad der Kieselsäurecluster größer als 50% ist.

2. Das Verfahren gemäß Anspruch 1, **dadurch gekennzeichnet, dass** das Sol durch Hydrolyse von Tetraethylorthosilicat (TEOS) erzeugt wird,
wobei bevorzugt ein r_{W} Wert (Molverhältnis von Wasser zu TEOS) von 4 genutzt wird, und/oder wobei als Lösungsmittel bevorzugt Ethanol verwendet wird,
und/oder wobei als Katalysator für die Hydrolyse bevorzugt eine organische Säure, bevorzugt Oxalsäure genutzt wird,
und/oder wobei bevorzugt das Wasser dann durch ein organisches Lösungsmittel, bevorzugt Ethanol, ersetzt wird, wenn die gewünschte Größe der Kieselgelcluster durch Kondensation erreicht wurde, wobei bevorzugt die gewünschte Größe im Bereich von 0.5 nm bis 4 nm liegt.

3. Das Verfahren gemäß Anspruch 1, **dadurch gekennzeichnet, dass** das Sol durch Ionenaustausch von Natriumwasserglaslösung erzeugt wird, wobei bevorzugt das Wasser dann durch ein organisches Lösungsmittel, bevorzugt Ethanol, ersetzt wird, wenn die gewünschte Größe der Kieselgelcluster durch Kondensation erreicht wurde, wobei bevorzugt die gewünschte Größe im Bereich von 0.5 nm bis 4 nm liegt.

4. Das Verfahren gemäß einem der vorhergehenden Ansprüche, wobei die Veresterung bevorzugt dadurch realisiert wird, indem bei Anwesenheit einer Säure, bevorzugt einer organischen Säure, besonders bevorzugt Oxalsäure, das Wasser, das bei der Veresterung entsteht, entfernt wird.

5. Das Verfahren gemäß einem der vorhergehenden Ansprüche, **dadurch gekennzeichnet,**
a) **dass** man einen Katalysator verwendet, wobei der verwendete Katalysator ausgewählt ist aus der Gruppe umfassend eine organische Säure, mehr bevorzugt Oxalsäure, und einen sauren Ionenaustauscher, bevorzugt sulfoniertes Polystyrol R-SO₃H; und/oder
b) **dass** das Sol einem Reifungsprozess unterzogen wird, bevor die SisurfaceOH- Gruppen verestert werden, wobei das Lösungsmittel Wasser oder ein Gemisch aus Wasser und organischem Lösungsmittel ist und ein pH-Wert von 7 oder mehr eingestellt wird; und/oder
c) **dass** die organischen Bestandteile oxidiert werden, wobei bevorzugt ein Sauerstoffplasma genutzt wird; und/oder
d) **dass** das Lösungsmittel entfernt wird, wobei bevorzugt ein Sprühtrocknungsverfahren verwendet wird.

6. Das Verfahren gemäß einem der vorhergehenden Ansprüche, **dadurch gekennzeichnet,**
a) **dass** das im Sol enthaltene Wasser durch ein organisches Lösungsmittel ersetzt wird, wenn die Kieselgelcluster eine Größe von 0.5 bis 4 nm haben,
wobei bevorzugt das Lösungsmittel Ethanol ist, und
wobei bevorzugt eine Veresterung durchgeführt wird, wobei bevorzugt das veresterte Sol soweit eingekocht wird, dass der Feststoffgehalt (SiO₂) im Sol bevorzugt im Bereich von 3 bis 25 Gew. % liegt und/oder
wobei bevorzugt das Sol sprühgetrocket wird, wobei die Sprühparameter bevorzugt so gewählt werden, dass hohle Mikropartikel entstehen; und/oder
b) **dass** das Lösungsmittel soweit entfernt wird, dass die Viskosität des Sols im Bereich von 0.1 Pas bis 10 Pas, bevorzugt im Bereich von 0.3 Pas bis 0.7 Pas liegt, und wobei bevorzugt dieses Sol zu Fäden gesponnen wird; und/oder
c) **dass** das im Sol enthaltene Wasser durch ein organisches Lösungsmittel ersetzt wird, wenn die Kieselgelcluster eine Größe von 0.5 bis 4 nm haben, wobei das Lösungsmittel bevorzugt Ethanol ist, dass eine Veresterung mit der Ethylgruppe durchgeführt wird, und dass bevorzugt das veresterte Sol soweit eingekocht wird, dass das Sol eine Viskosität bevorzugt im Bereich von 0.3 Pas bis 0.7 Pas hat und dass dieses Sol bevorzugt zu Gelfäden gesponnen wird, und/oder
d) **dass** durch Entfernen oder Hinzufügen von Lösungsmittel ein Feststoffgehalt (SiO₂) im Sol erhalten wird, der im Bereich von 3 bis 25 Gew.% liegt, wobei bevorzugt dieses Sol für bekannte Beschichtungsverfahren wie *dip coating, spin coating* oder *spray coating* genutzt wird.

7. Das Verfahren gemäß einem der vorhergehenden Ansprüche, **dadurch gekennzeichnet, dass** die gering vernetzten Kieselsäurestrukturen in eine wässrige Lösung eines Polymers, ausgewählt aus einer Gruppe umfassend Polyvinylpyrrolidon und Kollagen, gegeben werden,
wobei bevorzugt der Polymeranteil in der Lösung zwischen 2 und 15 Gew.% liegt und/oder
wobei bevorzugt der Anteil des SiO₂ in Bezug zur Polymer/Wasserlösung im Bereich von 2 bis 40 Gew.% liegt, und/oder
wobei bevorzugt die Lösung mit den homogen vermischten Kieselsäurestrukturen in eine Form gegossen und gefriergetrocknet wird.

8. Ein Biomaterial, erhältlich nach einem Verfahren nach einem der vorhergehenden Ansprüche, welches aus SiO₂ Polyederstrukturen aufgebaut ist, die eine Größe von 0.5 nm bis 4 nm aufweisen, die an der Oberfläche Si-O-R-Gruppen aufweisen, wobei R ein organischer Rest, bevorzugt eine Alkyl- oder Arylgruppe mit 1-10 C-Atomen, ausgewählt aus der Gruppe umfassend Methyl, Ethyl oder Phenyl, ist.

9. Das Biomaterial nach Anspruch 8, **dadurch gekennzeichnet, dass** es in einer Form vorliegt, die ausgewählt ist aus der Gruppe bestehend aus
- Granulat,
- Mikroteilchen, bevorzugt hohle Mikroteilchen, bevorzugt mit einem Durchmesser im Be reich von 2µm bis 100µm,
- Faden, bevorzugt mit einem Durchmesser im Bereich von 1 µm bis 50 µm,
- Schicht, wobei die Schicht bevorzugt eine Schicht auf einem Implantat oder einer Wundauflage ist.

10. Das Biomaterial nach Anspruch 8, **dadurch gekennzeichnet, dass** es in ein Trägermaterial eingebettet ist, wobei das Trägermaterial bevorzugt eine organische Membran, ein organisches Vlies oder ein organischer Schwamm ist, und/oder wobei das Trägermaterial bevorzugt ein biodegradierbares organisches Material ist, wobei als Träger bevorzugt ein Gelatineschwamm oder ein Schwamm aus Polyvinylpyrrolidon verwendet wird.

11. Das Biomaterial nach einem der Ansprüche 8 bis 10 zur Verwendung
a) als Medizinprodukt, das eine stützende oder abschirmende Funktion hat und bevorzugt gleichzeitig durch die Degradation als Lieferant für das die Geweberegeneration unterstützende Siliziumdioxid dienen kann, oder
b) für die Behandlung von Wunden oder Narben oder für kosmetische Anwendungen, insbesondere als Salbe oder Creme.

12. Ein Medizinprodukt oder ein Nahrungsergänzungsmittel, umfassend das Biomaterial nach einem der Ansprüche 8 bis 10.

## Claims

1. Method for preparing silicic acid structures having a low degree of crosslinking, said method comprising:
a) producing a sol,
b) replacing the water contained in the sol by a water-soluble organic solvent,
c) after elimination of the water by an organic solvent, esterifying the Si_{surface}OH groups present in the sol on the internal surface of the silicic acid clusters to Si_{surface}OR, wherein R is an organic rest, wherein R is an alkyl or aryl group selected from the group consisting of methyl, ethyl, propyl, butyl, pentyl, hexyl, and phenyl, and wherein the degree of esterification of the silicic acid clusters is greater than 50%.

2. The method according to claim 1, wherein the sol is produced by hydrolysis of tetraethyl orthosilicate (TEOS),
wherein an r_{w} value (molar ratio of water to TEOS) of 4 is preferably used,
and/or wherein ethanol is preferably used as the solvent,
and/or wherein an organic acid, preferably oxalic acid, is preferably used as the catalyst for the hydrolysis,
and/or wherein the water is preferably replaced by an organic solvent, preferably ethanol, when the desired size of the silica gel clusters has been achieved by condensation, wherein the desired size preferably is in the range from 0.5 nm to 4 nm.

3. The method according to claim 1, wherein the sol is produced by ion exchange of sodium water glass solution, wherein the water is preferably replaced by an organic solvent, preferably ethanol, when the desired size of the silica gel clusters has been achieved by condensation, wherein the desired size preferably is in the range of 0.5 nm to 4 nm.

4. The process according to any of the preceding claims, wherein the esterification is preferably achieved by removing the water formed upon esterification in the presence of an acid, preferably an organic acid, more preferably oxalic acid.

5. The method according to any of the preceding claims, wherein
a) a catalyst is used, wherein the catalyst used is selected from the group comprising an organic acid, more preferably oxalic acid, and an acidic ion exchanger, preferably sulfonated polystyrene R-SO₃H; and/or
b) the sol is subjected to a maturation process before the Si_{surface}OH groups are esterified, wherein the solvent is water or a mixture of water and an organic solvent, and a pH of 7 or more is adjusted; and/or
c) the organic components are oxidized, wherein preferably an oxygen plasma is used; and/or
d) the solvent is removed, wherein preferably a spray-drying process is used.

6. The method according to any of the preceding claims, wherein
a) the water contained in the sol is replaced by an organic solvent when the silica gel clusters have a size of 0.5 to 4 nm, wherein the solvent preferably is ethanol, and wherein preferably an esterification is carried out, wherein the esterified sol preferably is boiled down to such an extent that the solids content (SiO₂) in the sol preferably is in the range from 3 to 25% by weight and/or wherein the sol is preferably spray-dried, wherein the spray parameters are preferably selected such that hollow microparticles are formed; and/or
b) the solvent is removed to such an extent that the viscosity of the sol is in the range of 0.1 Pas to 10 Pas, preferably in the range of 0.3 Pas to 0.7 Pas, and wherein said sol is preferably spun into filaments; and/or
c) the water contained in the sol is replaced by an organic solvent when the silica gel clusters have a size of 0.5 to 4 nm, wherein the solvent is preferably ethanol, and wherein esterification with the ethyl group is carried out, and wherein the esterified sol is preferably boiled down to such an extent that the sol preferably has a viscosity in the range from 0.3 Pas to 0.7 Pas and wherein said sol is preferably spun into gel filaments; and/or
d) a solids content (SiO₂) in the sol in the range from 3 to 25% by weight is achieved by removal or addition of solvent, wherein said sol is preferably used for known coating methods such as dip coating, spin coating or spray coating.

7. The method according to any of the preceding claims, wherein the low-crosslinked silicic acid structures are added to an aqueous solution of a polymer selected from a group comprising polyvinylpyrrolidone and collagen, wherein the polymer content in the solution preferably is between 2 and 15% by weight; and/or wherein the proportion of SiO₂ with respect to the polymer/water solution preferably is in the range of 2 to 40% by weight, and/or wherein the solution is preferably poured with the homogeneously mixed silicic acid structures into a mould and freeze-dried.

8. A biomaterial obtainable by a process according to any of the preceding claims, which is composed of SiO₂ polyhedral structures having a size of 0.5 nm to 4 nm having Si-O-R groups on the surface, wherein R is an organic rest, preferably an alkyl or aryl group having 1-10 C atoms selected from the group comprising methyl, ethyl or phenyl.

9. The biomaterial according to claim 8, wherein said biomaterial is present in a form selected from the group consisting of
- granules,
- microparticles, preferably hollow microparticles, preferably with a diameter in the range from 2 µm to 100 µm,
- thread, preferably with a diameter in the range of 1 µm to 50 µm,
- layer, wherein the layer preferably is a layer on an implant or wound dressing.

10. The biomaterial according to claim 8, wherein said biomaterial is embedded in a carrier material, wherein the carrier material preferably is an organic membrane, an organic non-woven material or an organic sponge, and/or wherein the carrier material preferably is a biodegradable organic material, and wherein a gelatin sponge or a polyvinylpyrrolidone sponge is preferably used as a carrier.

11. The biomaterial according to any of claims 8 to 10 for use
a) as a medical device which has a supporting or shielding function and at the same time preferably serves by degradation as a supply for silica which assists tissue regeneration, or
b) for the treatment of wounds or scars or for cosmetic applications, in particular as an ointment or cream.

12. A medical device or dietary supplement comprising the biomaterial of any of claims 8 to 10.

## Revendications

1. Procédé de production de structures d'acide silicique peu réticulées, dans lequel
a) on prépare un sol,
b) on remplace l'eau contenue dans le sol par un solvant organique hydrosoluble,
c) et une fois l'eau éliminée à l'aide d'un solvant organique, on estérifie les groupes Si*_{surface}*-OH, présents dans le sol à la surface interne des clusters d'acide silicique, en groupes Si*_{surface}*-OR où R représente un groupe organique, étant entendu que R représente un groupe alkyle ou aryle, choisi dans l'ensemble constitué par les groupes méthyle, éthyle, propyle, butyle, pentyle, hexyle et phényle, et étant entendu que le degré d'estérification des clusters d'acide silicique est supérieur à 50 %.

2. Procédé conforme à la revendication 1, **caractérisé en ce que** l'on prépare le sol par hydrolyse d'orthosilicate de tétraéthyle (OSTE), étant entendu que :
- de préférence, on utilise de l'eau en un rapport molaire r_{w} de l'eau à l'OSTE valant 4,
- et/ou, de préférence, on utilise de l'éthanol comme solvant,
- et/ou, de préférence, on utilise un acide organique comme catalyseur pour l'hydrolyse, en particulier de l'acide oxalique,
- et/ou, de préférence, on remplace l'eau par un solvant organique, en particulier de l'éthanol, quand les clusters d'acide silicique ont atteint par condensation la taille voulue, cette taille voulue se situant de préférence dans l'intervalle allant de 0,5 nm à 4 nm.

3. Procédé conforme à la revendication 1, **caractérisé en ce que** l'on prépare le sol en opérant un échange d'ions dans une solution de silicate de sodium (verre soluble), étant entendu que, de préférence, on remplace l'eau par un solvant organique, en particulier de l'éthanol, quand les clusters d'acide silicique ont atteint par condensation la taille voulue, cette taille voulue se situant de préférence dans l'intervalle allant de 0,5 nm à 4 nm.

4. Procédé conforme à l'une des revendications précédentes, dans lequel on opère de préférence l'estérification en éliminant l'eau formée lors de l'estérification en présence d'un acide, qui est de préférence un acide organique, et en particulier de l'acide oxalique.

5. Procédé conforme à l'une des revendications précédentes, **caractérisé en ce que**
a) on utilise un catalyseur, qui est choisi dans l'ensemble comprenant un acide organique, de préférence l'acide oxalique, et un échangeur d'ions acide, de préférence un polystyrène sulfoné R-SO₃H,
b) et/ou avant que les groupes Si*_{surface}*-OH soient estérifiés, on fait subir au sol un processus de mûrissement, où le solvant est de l'eau ou un mélange d'eau et de solvant organique et le pH est ajusté à une valeur de 7 ou plus,
c) et/ou l'on oxyde les constituants organiques, de préférence en utilisant un plasma d'oxygène,
d) et/ou l'on élimine le solvant, de préférence en opérant un séchage par atomisation.

6. Procédé conforme à l'une des revendications précédentes, **caractérisé en ce que**
a) l'on remplace l'eau contenue dans le sol par un solvant organique quand les clusters de gel de silice ont une taille de 0,5 à 4 nm, étant entendu
- que, de préférence, le solvant est de l'éthanol,
- et que, de préférence, l'on opère une estérification, le sol estérifié étant alors, de préférence, concentré par ébullition à tel point que la teneur en solides (SiO₂) du sol se situe de préférence dans l'intervalle allant de 3 à 25 % en poids,
- et/ou que, de préférence, le sol est séché par atomisation,
les paramètres d'atomisation étant alors, de préférence, choisis de telle sorte qu'il se forme des microparticules creuses ;
b) et/ou l'on élimine le solvant à tel point que la viscosité du sol se situe dans l'intervalle allant de 0,1 à 10 Pa.s, et de préférence dans celui allant de 0,3 à 0,7 Pa.s, étant entendu que, de préférence,
ce sol est filé et réduit à l'état de fils ;
c) et/ou l'on remplace l'eau contenue dans le sol par un solvant organique quand les clusters de gel de silice ont une taille de 0,5 à 4 nm, étant entendu que ce solvant est de préférence de l'éthanol, on opère une estérification avec le groupe éthyle,
et de préférence, on concentre le sol estérifié, à tel point que le sol présente de préférence une viscosité située dans l'intervalle allant de 0,3 à 0,7 Pa.s, et ce sol est de préférence filé et réduit à l'état de fils de gel ;
d) et/ou, en chassant du solvant ou en en ajoutant, l'on ajuste la teneur en solides (SiO₂) du sol à une valeur située dans l'intervalle allant de 3 à 25 % en poids, ce sol étant utilisé de préférence dans des procédés de revêtement connus, comme le revêtement par immersion (*dip coating*)*,* le revêtement par rotation (*spin coating*) ou le revêtement par projection (*spray coating*)*.*

7. Procédé conforme à l'une des revendications précédentes, **caractérisé en ce que** les structures d'acide silicique peu réticulées sont produites dans une solution aqueuse d'un polymère choisi dans un ensemble comprenant un poly(vinyl-pyrrolidone) et un collagène, étant entendu
- que, de préférence, la proportion de polymère dans la solution se situe entre 2 et 15 % en poids,
- et/ou que, de préférence, la proportion de SiO₂, par rapport à la solution aqueuse de polymère, se situe dans l'intervalle allant de 2 à 40 % en poids,
- et/ou que, de préférence, on verse dans un moule la solution comprenant, en un mélange homogène, les structures d'acide silicique, et on la lyophilise.

8. Biomatériau, accessible par un procédé conforme à l'une des revendications précédentes, qui est formé de structures en polyèdres de SiO₂ présentant une taille de 0,5 à 4 nm, ainsi que, à leur surface, des groupes de formule Si-O-R où R représente un groupe organique, de préférence un groupe alkyle ou aryle comportant de 1 à 10 atomes de carbone, choisi dans l'ensemble comprenant les groupes méthyle, éthyle et phényle.

9. Biomatériau conforme à la revendication 8, **caractérisé en ce qu'**il se présente sous une forme choisie dans l'ensemble constitué par les suivantes :
- un granulat,
- des microparticules, de préférence creuses, et présentant de préférence un diamètre situé dans l'intervalle allant de 2 à 100 µm,
- des fils, présentant de préférence un diamètre situé dans l'intervalle allant de 1 à 50 µm,
- une couche, laquelle couche est de préférence une couche placée sur un implant ou sur un pansement.

10. Biomatériau conforme à la revendication 8, **caractérisé en ce qu'**il est enrobé dans un matériau support, lequel matériau support est de préférence une membrane organique, un non-tissé organique ou une éponge organique, et/ou lequel matériau support est de préférence un matériau organique biodégradable, étant entendu que de préférence, on utilise comme support une éponge en gélatine ou une éponge en poly(vinyl-pyrrolidone).

11. Biomatériau, conforme à l'une des revendication 8 à 10, pour utilisation
a) en tant que produit médical qui joue un rôle de soutien ou
de protection et qui, de préférence, peut servir en même temps,
en se dégradant, de pourvoyeur de dioxyde de silicium aidant à la régénération d'un ou de tissu(s),
b) ou dans le traitement de blessures ou de cicatrices ou dans des applications cosmétiques, en particulier sous forme de pommade ou de crème.

12. Produit médical ou complément alimentaire, comprenant un biomatériau conforme à l'une des revendications 8 à 10.
